(19) Europäisches Patentamt
European Patent Office
Office européen des brevets

(11) **EP 4 037 469 B1**

(12) **EUROPEAN PATENT SPECIFICATION**

(45) Date of publication and mention
of the grant of the patent:
**15.04.2026 Bulletin 2026/16**

(21) Application number: **20775895.4**

(22) Date of filing: **29.09.2020**

(51) International Patent Classification (IPC):
**C12N 1/20** (2026.01)

(52) Cooperative Patent Classification (CPC):
**F25C 3/04; C07K 14/21; C12N 1/20; A01G 15/00**

(86) International application number:
**PCT/EP2020/077224**

(87) International publication number:
**WO 2021/063943 (08.04.2021 Gazette 2021/14)**

(54) **BACTERIAL PREPARATIONS FOR ICE NUCLEATION**

BAKTERIELLE PRÄPARATE ZUR EISNUKLEATION

PRÉPARATIONS BACTÉRIENNES POUR LA NUCLÉATION DE LA GLACE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.09.2019 EP 19200540**

(43) Date of publication of application:
**10.08.2022 Bulletin 2022/32**

(73) Proprietor: **Logon Patentverwertungs-Verwaltungs GmbH**
**87527 Ofterschwang (DE)**

(72) Inventors:
  • **LUBITZ, Werner**
    **3420 Klosterneuburg - Kritzendorf (AT)**
  • **RESCH, Mascha**
    **1140 Wien (AT)**
  • **KASSMANNHUBER, Johannes**
    **6850 Dornbirn (AT)**

(74) Representative: **Weickmann & Weickmann PartmbB**
    **Postfach 860 820**
    **81635 München (DE)**

(56) References cited:
    **EP-A1- 1 829 890**

• **PANKOVA IVETA: "Serological and biochemical distinguishing of Pseudomonas syringae: Pathovars on peas", PLANT PROTECTION SCIENCE, vol. 35, no. 3, 1 January 1999 (1999-01-01), pages 79 - 84, XP093262285**
• **JOHANNES KASSMANNHUBER ET AL: "Freezing from the inside. Ice nucleation in Escherichia coli and Escherichia coli ghosts by inner membrane bound ice nucleation protein InaZ", 15 January 2019 (2019-01-15), XP055750925, Retrieved from the Internet <URL:https://acp.copernicus.org/preprints/acp-2019-23/> [retrieved on 20201116], DOI: 10.5194/acp-2019-23**
• **KASSMANNHUBER JOHANNES ET AL: "Functional display of ice nucleation protein InaZ on the surface of bacterial ghosts", BIOENGINEERED, vol. 8, no. 5, 2017, pages 488 - 500, XP002801138**
• **JANET S. H. LORV ET AL: "Bacterial Ice Crystal Controlling Proteins", SCIENTIFICA, vol. 2014, 1 January 2014 (2014-01-01), pages 1 - 20, XP055751137, DOI: 10.1155/2014/976895**

Remarks:
The complete document including Reference Table(s) and the Sequence Listing(s) can be downloaded from the EPO website

**Description**

The present invention relates to bacterial preparations such as inactivated bacteria or bacterial ghosts comprising ice nucleation proteins. Such bacterial preparations can in particular be used for the production of snow or rain, for hail suppression or for fog dispersal.

Ice nucleation proteins (INP) of ice nucleation active bacteria have the property to catalyze heterogeneous ice formation of water and are used, for example, for snowmaking in ski resorts using snow cannons. By using such ice nucleation proteins the water and energy requirement for producing snow can be reduced.

In nature, ice nucleation proteins (INP) expressed by bacteria are localized on the outer membrane of the bacteria. The biological function of INPs is to direct ice formation into the extracellular space at warm sub-zero temperatures to provide adaption time for the bacteria to freezing stress. Furthermore, the resulting osmotic imbalance due to the extracellular ice formation results in an above-average water outflow from the cell to lower the intracellular ice nucleation (IN) temperature.

J. Kassmannhuber et al., Atmos. Chem. Phys. Discuss., DOI: 10.5194/acp-2019-23 refers to ice nucleation in Escherichia coli and Escherichia coli ghosts by inner membrane bound ice nucleation protein InaZ.

J. Kassmannhuber et al., Bioengineered, vol. 8, no. 5, 2017, p 488-500 describes functional display of ice nucleation protein InaZ on the surface of bacterial ghosts.

J. S. H. Lorv et al., Scientifica, vol. 2014, p 1-20 is a review article of bacterial ice crystal controlling proteins.

A problem to be solved by the present invention was to provide an improved ice nucleation active composition.

According to a first aspect, the present invention relates to a bacterial preparation comprising inactivated bacteria, bacterial ghosts (BGs) or fragments thereof which carry an ice nucleation protein (INP) being the ice nucleation protein Z (InaZ) of Pseudomonas syringae and which are fixed with the fixation agent glutaraldehyde, wherein the bacterial preparation comprises inactivated bacteria, wherein the ice nucleation protein (INP) is within the cytoplasm and/or comprises bacterial ghosts (BGs) from Gram-negative bacteria which carry the ice nucleation protein (INP) anchored at the outer membrane (OM); and wherein the ice nucleation protein (INP) being the ice nucleation protein Z (InaZ) of Pseudomonas syringae is heterologous to the inactivated bacteria or bacterial ghosts (BGs).

Surprisingly, it has been found that excellent ice nucleation activity can be achieved by fixing bacterial preparations comprising inactivated bacteria, bacterial ghosts (BGs) or fragments thereof, which carry an ice nucleation protein (INP), with a fixation agent. For example, in a droplet freezing assay (DFA), such bacterial preparations show formation of ice crystals already at relatively high temperatures such as, for example, from -4°C to -7°C.

Without wishing to be bound to a theory, it is assumed that treatment with a fixation agent leads to the formation of ordered or arrayed structures serving as an ice formation nucleus.

According to the invention glutaraldehyde is used as fixation agent. Particularly good results are achieved when the fixation agent is glutaraldehyde (also termed 1,5-pentanedial and also abbreviated as GA herein). Applying glutaraldehyde results in fixing of INP structures carried on inactivated bacteria, bacterial ghosts or fragments thereof, providing a starting point for ice formation. At the same time, glutaraldehyde can serve to inactivate bacteria.

Suitably, glutaraldehyde is applied in an amount of at least 0.02%, more preferably of at least 0.05%, even more preferably of at least 0.1% and still more preferably of at least 0.2% and up to 2%, more preferably up to 1% (all percentages refer to vol./vol. based on the treated bacterial or BG suspension). The inactivation/fixation with glutaraldehyde is performed, in particular, by incubation of bacteria or bacterial ghosts containing suspensions, in particular, suspensions containing from $0.1 \times 10^{10}$ bacteria or bacterial ghosts up to $10 \times 10^{10}$ bacteria or bacterial ghosts per ml. Incubation/fixation with glutaraldehyde is performed preferably for at least 5 minutes, more preferably at least 10 minutes, and up to 60 minutes, more preferably up to 50 minutes. While short incubation times usually are sufficient, it is also possible to incubate bacteria with glutaraldehyde for up to 48 hours, more preferably for up to 24 hours, and most preferably for up to 12 hours, e.g. overnight. Inactivation/fixation with glutaraldehyde preferably can be performed at room temperature, i.e. at temperatures from 20°C up to 25°C.

After incubation with glutaraldehyde the obtained bacterial preparations are freed from glutaraldehyde and washed in a suitable manner.

Surprisingly, it was found that bacterial preparations comprising inactivated bacteria, bacterial ghosts (BGs) or fragments thereof show excellent ice nucleation activity even if they have been treated with glutaraldehyde. This is surprising all the more since other activities such as, for example, RNase activity of membrane-bound RNase are inhibited by treatment with glutaraldehyde. Conventionally, it was assumed that any activity is inactivated by treatment with glutaraldehyde. In contrast to this assumption, it turned out that an excellent ice nucleation activity can be seen in the inventive bacterial preparations even after treatment with glutaraldehyde. This is attributed to the fact that the inventive activity is caused by the formation of ordered or arrayed structures which then serve as an ice formation nucleus.

The bacterial preparation comprises inactivated bacteria, wherein the ice nucleation protein (INP) is within the cytoplasm, and/or comprises bacterial ghosts (BGs) from Gram-negative bacteria which carry the ice nucleation protein (INP) anchored at the outer membrane (OM). Particularly preferred are bacterial preparations comprising bacterial ghosts (BGs) or fragments thereof which carry an ice nucleation protein (INP) anchored at the outer membrane.

Unless otherwise indicated, the following definitions apply to all embodiments described herein:
Ice nucleation: The mechanism of ice nucleation (IN) is generally divided into homogeneous and heterogeneous nucleation.

Homogeneous IN occurs without any foreign substance aiding the process of ice formation and homogeneous freezing of ultra-pure water occurs only at highest supercooled and supersaturated conditions, e.g. for atmospheric water at temperatures below -38°C.

Heterogeneous freezing is defined as IN aided by the presence of a foreign substance such as bacteria or others so that nucleation takes place at a lesser supersaturation or supercooling than is required for homogeneous IN. For homogeneous ice formation at -5°C, 45,000 water molecules are required in an embryo ice crystal though this number can drop to 600 or lower when ice nuclei are present. So-called ice nuclei trigger ice formation at temperatures between 0°C and -35°C. Heterogeneous IN is subdivided into four modes all of which can be initiated by INP-Bacterial preparations.

(1) Immersion freezing, where the ice nuclei are incorporated in a liquid body and initiate nucleation from inside the droplet.
(2) Condensation freezing, where water droplets are formed by condensation around a cloud condensation nucleus (CCN), which acts simultaneously as ice nuclei.
(3) Contact freezing is caused by ice nuclei upon contact with a droplet surface.
(4) Deposition freezing where IN occurs directly from water vapor upon an ice nuclei surface.

Ice nucleation protein (INP): An ice nucleation protein is a protein which promotes the formation of ice from water. Water molecules are stepwise aggregated by electrostatic attraction of the polar parts of water by INPs until the critical size is exceeded to form (embryo) ice crystals.

Preferred ice nucleation proteins are bacterial ice nucleation proteins, preferably from Gram-negative bacteria, which can act as an ice nucleus. Particularly suitable are ice nucleation proteins from Pseudomonas syringae, Pseudomonas putida, Erwinia herbicola, Erwinia ananas, or Xanthomonas campestris. The ice nucleation protein of Pseudomonas syringae is particularly preferred. According to the invention, the ice nucleation protein (INP) is the ice nucleation protein Z (InaZ) of Pseudomonas syringae.

The plant pathogen P. syringae represents one of the most efficient bacterial ice nucleus, initiating plant damaging ice formation at temperatures starting at -2°C. INPs have the property to catalyze heterogeneous ice formation of supercooled water by orienting water molecules into an ice-like structure.

Bacterial INPs are mostly composed of three characteristic protein domains, a highly repetitive central domain with several tandem consensus octapeptides (AGYGSTLT) and non-repetitive N- and C-terminal domains. The relatively hydrophobic N-terminal domain, which is usually approximately 15% of the protein, is assumed to bind to phosphatidylinositol and polysaccharides and functions as outer membrane anchor. The central domain is predicted to form beta-helical structures mimicking an ice-like surface, which is able to bind water molecules and functions as a template for orientating water into a lattice structure. The function of the hydrophilic C-terminal domain is believed to have an important role in stabilizing the INP conformation.

The INPs employed in the present invention are preferably proteins comprising at least one, preferably at least two and more preferably at least three sequence segments AGYGSTLT.

The bacterial preparations of the present invention can comprise inactivated bacteria, bacterial ghosts, fragments of inactivated bacteria or fragments of bacterial ghosts.

Inactivated bacteria are bacteria which are no longer capable of reproduction. Inactivated bacteria can be prepared by incubating bacteria with an inactivating agent, such as β-propiolactone. In a preferred embodiment according to the present invention, inactivation and at the same time fixation is done by incubation with glutaraldehyde.

Preferably, the inactivated bacteria of the present invention are derived from E. coli, in particular, E. coli POP2135, E. coli C41 or E. coli K12.

Bacterial Ghosts (BGs) are empty, non-living particles, which can be generated from bacteria, in particular from Gram-negative bacteria and most preferably from E.coli through a process whereby the cell content is ejected through a hole formed in the cell envelope complex, resulting in an empty cell envelope. Although being non-living and non-infectious BGs have all external properties which are characteristic for the living bacteria. In particular, bacterial ghosts (BGs) are empty cell envelopes of Gram-negative bacteria, which can be produced by the expression of clone gene E of bacteriophage PhiX 174 (E. Henrich et al., Mol. Gen. Genet. 185 (3) (1982), 493-497), which forms a tunnel structure through the bacterial inner membrane (IM) and outer membrane (OM). The cytoplasmic content of the bacterium is expelled through this tunnel. The BG internal lumen is free of nucleic acids, ribosomes and other constituents, while the inner and outer membrane structures of the cell envelope are well preserved.

Preferably, the bacterial ghost (BG) of the invention is an E.coli bacterial ghost and in particular an E. coli POP2135, an E.coli C41 or an E. coli K12 bacterial ghost.

Fragments of inactivated bacteria according to the present invention preferably have a size (diameter) of from 70 to 100 nm. Those fragments can be prepared from bacteria or from inactivated bacteria, e.g. using an atomizer.

Fragments of bacterial ghosts according to the present invention preferably have a size (diameter) of from 70 to 100 nm. Such fragments can be prepared from bacterial ghosts, e.g. using an atomizer.

Preferably, the present invention relates to a bacterial preparation comprising an ice nucleation protein (INP) lacking a transport sequence for localization in the outer membrane.

It was surprisingly found that such non-naturally occurring bacterial preparations have excellent ice nucleation activity.

Due to the use of an ice nucleation protein lacking a transport sequence for localization in the outer membrane the bacterial preparations of the invention preferably comprise the ice nucleation protein in the cytoplasm. It is surprising that such bacterial preparations show ice nucleation activity. The general mechanism of bacteria expressing ice nucleation proteins is that they express the INPs on the outside, causing damage of the plant, resulting in leakage of plant sap.

Contrary to these favorable conditions for bacteria, freezing from inside in natural environment would result in inactivation and killing of the bacteria. Nevertheless, the inventors of the present application have found that ice nucleation proteins can be expressed and localized at the inside of bacteria, in particular in the cytoplasm, resulting in efficient ice nucleation active compositions.

In this preferred embodiment, the inventive bacterial preparation comprises an ice nucleation protein (INP) lacking a transport sequence for localization in the outer membrane. The transport sequence for localization in the outer membrane which is present in naturally occurring INPs is completely or partially truncated and/or inactivated, thereby preventing arrangement of the INP in the outer membrane of the bacterial preparation.

In the INPs used in this embodiment of the present invention, in particular the N-terminal domain is completely or partially truncated. Thus, the INPs do not have an intact, functioning transport sequence for localization in the outer membrane.

According to the invention, the INP is from Pseudomonas syringae, namely the ice nucleation protein Z (InaZ) from Pseudomonas syringae. In a particularly preferred embodiment, the ice nucleation protein is a N-terminal truncated form of the ice nucleation protein (INP) of Pseudomonas syringae. Particularly preferred is a N-terminal truncated form of InaZ, which is lacking the transporter sequence for the localization of InaZ in the outer membrane.

A membrane-targeting system is based on the hydrophobic membrane-spanning domains of the truncated E(E') and L(L') proteins of the bacteriophages ΦX174 and SM2, respectively, that localize foreign proteins to the inner membrane of the cell envelope (Szostak et al., Research in Microbiology, 141 (7-8) (1990), 1005-1007). The ice nucleation protein can be anchored to the inner membrane via amino-terminal E' sequence, via carboxy-terminal L' sequence or with both sequences.

Most preferred is a truncated N-terminal form of InaZ ice nucleation protein (INP) of Pseudomonas syringae comprising the amino acid sequence shown in SEQ ID NO: 1 or at least a section thereof having at least 100 amino acids, more preferably having at least 120 amino acids.

A further preferred embodiment of the inventive bacterial preparation is a bacterial ghost (BG), which comprises an ice nucleation protein (INP) lacking a transport sequence for localization in the outer membrane.

Bacterial ghosts of an embodiment of the invention comprise an ice nucleation protein (INP) lacking a transport sequence for localization in the outer membrane.

It is possible to express an ice nucleation protein (INP), preferably in Gram-negative bacteria such as E.coli and then further process the bacteria to bacterial ghosts. However, it is also possible to first produce bacterial ghosts and then immobilize an ice nucleation protein at the inner membrane of the formed bacterial ghost.

A further preferred embodiment of the pesent invention is a bacterial preparation comprising inactive bacteria, which comprise an ice nucleation protein (INP) in the cytoplasm.

Surprisingly, it was found that the inactivated bacteria of the invention have a high ice nucleation (IN) activity catalyzing ice formation at high sub-zero temperaturs.

In nature, bacterial ice nucleation proteins (INPs) bound to the outer membrane of the some gram-negative bacteria can act as an ice nucleus, for example in Pseudomonas syringae, Pseudomonas putida, Erwinia herbicola, Erwinia ananas or Xanthomonas campestris. INPs have the property to catalyze heterogeneous ice formation by orienting water molecules into an ice-like structure. The natural biological function of INPs is sought to direct ice formation into the extracellular space at warm sub-zero temperatures to provide adaptation time for the bacterium to freezing stress. Furthermore, the resulting osmotic imbalance due to the extracellular ice formation results in an above-average water outflow from the cell to lower the intracellular IN temperature.

In contrast thereto, freezing from the inside of the cytoplasmic space is artificial and evidently does not provide any advantage for the survival strategy of bacteria at low temperatures. Surprisingly, the bacterial preparations of the invention comprising an ice nucleation protein in the cytoplasmic space show good ice nucleation activity. Bacterial preparations of the invention can be prepared by expression of truncated forms of ice nucleation proteins such as InaZ of P. syringae, without a transport sequence for location in the outer membrane, which ice nucleation proteins are expressed in the cytoplasm, in particular of E. coli.

The inactivated bacterium of the invention is preferably an inactivated E. coli and in particular an inactivated E. coli POP2135, an inactivated E. coli C41 or an inactivated E. coli K.12. Preferably, inactivated bacteria according to the present invention are prepared by providing living bacteria comprising an ice nucleation protein within the cytoplasm and then inactivating said bacteria. Inactivation can be done e.g. using beta-propiolactone. Preferably, however, glutaraldehyde is used, by which bacteria, in particular E. coli, are inactivated and denatured and at the same time fixed. Due to its crosslinking properties, application of glutaraldehyde results in fixing of structures and in particular in fixing of β-sheets of ice nucleation proteins. It was found that bacteria, in particular E. coli, inactivated by glutaraldehyde show a further enhanced ice nucleation activity.

In a further preferred embodiment, the inventive bacterial preparation is a fragment of a bacterium, in particular E. coli, which comprises an ice nucleation protein (INP) in the cytoplasm.

It has been found that fragments of bacteria or of bacterial ghosts carrying an INP show ice nucleation activity and are in particular capable of functioning as cloud condensation seeds. Preferred are fragments having a size (diameter) of from 70 - 100 nm. Such fragments can be prepared from bacteria or bacterial ghosts using an atomizer.

The invention further relates to a bacterial preparation comprising inactivated bacteria, bacterial ghosts (BGs) or fragments thereof which carry a heterologous ice nucleation protein (INP). Such a bacterial preparation in particular comprises inactivated E. coli bacteria, E. coli bacterial ghosts (BGs) or fragments thereof which carry an ice nucleation protein (INP) of Pseudomonas syringae.

It was found that the inventive bacterial preparation and in particular the inventive inactivated bacteria and the inventive bacterial ghosts have a high ice nucleation activity. In particular, median freezing temperatures ($T_{50}$) of between -3°C and -10°C, preferably between -5°C and -9°C and in particular between -6°C and -8°C are achieved with the inventive bacterial preparations. This is a considerable increase in the median freezing temperatures compared to a median freezing temperature ($T_{50}$) of -20.1°C for living E.coli bacteria without INP and of -18.9°C for BGs not carrying INPs.

The inventive bacterial preparation and in particular the inventive bacterial ghosts or the inventive inactivated bacteria are in particular useful for snowmaking. In particular, the temperature at which artificial snow can be produced can be significantly increased compared to pure water.

Thus, the present invention is also directed to the use of bacterial preparations and in particular of bacterial ghosts as described herein for ice nucleation and in particular for the production of snow.

However, it is also possible to influence climatic factors by the inventive bacterial preparations and in particular by bacterial ghosts.

The inventive bacterial preparations and preferably bacterial ghosts can in particular be used for geo-engineering. The inventive bacterial preparations and preferably bacterial ghosts can in particular be used for weather modifications where they can find their application in heterologous ice nucleation for rain or snow production.

Therefore, the present invention is also directed to the use of bacterial preparations and preferably of bacterial ghosts as described herein for the production of rain, for cloud seeding, weather modification, as cloud condensation nuclei (CCN), for artificial cloud creation or for ice nucleation in the atmosphere.

Cloud seeding aims to induce rain in areas suffering from drought, to reduce the size of hailstones in storms and fog around airports, and also to make it snow at ski resorts. The procedure conventionally involves injecting silver iodide (historically also dry ice) into clouds. The iodide disperses into the clouds and forms ice crystals, which turn into rain drops falling from the clouds. Cloud seeding can be referred to as a weather modification process that helps in producing or inducing precipitation (rain or snow), depending upon the temperature conditions within the clouds. The dispersal of the particles may be done by an aircraft, a rocket, or even a ground seeding generator. The bacterial preparations and preferably the bacterial ghosts described herein can be used for cloud seeding and thus for rain and/or snow production.

The inventive bacterial preparations and preferably bacterial ghosts can also be used as cloud condensation nuclei (CCN). Good cloud condensation nuclei (CCN) must be small particles so that they do not settle too fast and they must be hydrophilic. The inventive bacterial preparations and preferably INP-BGs fulfil both criteria. The weight of bacterial ghosts is only about one quarter of the weight of an intact natural bacterium and the outer components of the bacterial ghosts are composed of hydrophilic proteins and sugar polymers. Thus, the inventive bacterial ghosts are small particles, which do not settle fast and are further hydrophilic, which characteristics make them perfect cloud condensation nuclei (CCN).

Further, the inventive bacterial preparations and preferably the inventive bacterial ghosts can be used for artificial cloud creation.

In case of cloudless areas where the induction of rain is planned none the less, cloud seeding is part of a three stage program. In the so-called agitation phase, conventionally chemicals such as calcium chloride, calcium carbide, calcium oxide, or urea and ammonium nitrate are used to stimulate the air mass upwind of the target area to rise and form rain clouds. During the buildup stage the cloud mass is built up using kitchen salt, urea, ammonium nitrate, dry ice, or calcium chloride to increase nuclei which also increase the density of the clouds. Finally, in the cloud seeding stage the created cloud is induced to rain down. Artificial rain creation is widely used in many countries throughout the world, such as the US, Chile, Venezuela, Cuba, China, and India.

Since current rain making technology is still not perfect and the chemicals used in the production of artificial rain could affect climatic patterns, ecosystem, contaminate water sources and the soil there is an urgent need on identifying new substances to be used for this purpose. The inventive bacterial preparations and preferably the bacterial ghosts address this need and can be applied in artificial cloud formation.

Further the inventive bacterial preparations and preferably the inventive bacterial ghosts can be used for hail suppression or for fog dispersal.

According to the invention it was found that the uses given herein, in particular production of rain, cloud seeding, weather modification, cloud condensation nuclei, artificial cloud creation as well as ice nucleation and also hail suppression and fog dispersal cannot only be caused by the inventive bacteria or bacterial ghosts, but also by fragments thereof. In particular, bacteria or bacterial ghosts comprising an ice nucleation protein anchored at the inner membrane can be fragmented using an atomizer. It has been seen that fragments having a size of 70 to 100 nm in diameter can particularly preferably be employed.

The invention is further explained by the enclosed Figures and the following examples.

Figure 1 shows schematically an arrangement of INP on the outer membrane of bacteria or of bacterial ghosts (Figure 1A) as well as an arrangement, wherein INPs are anchored at the inner membrane of a bacterium or a bacterial ghost (Figure 1B).

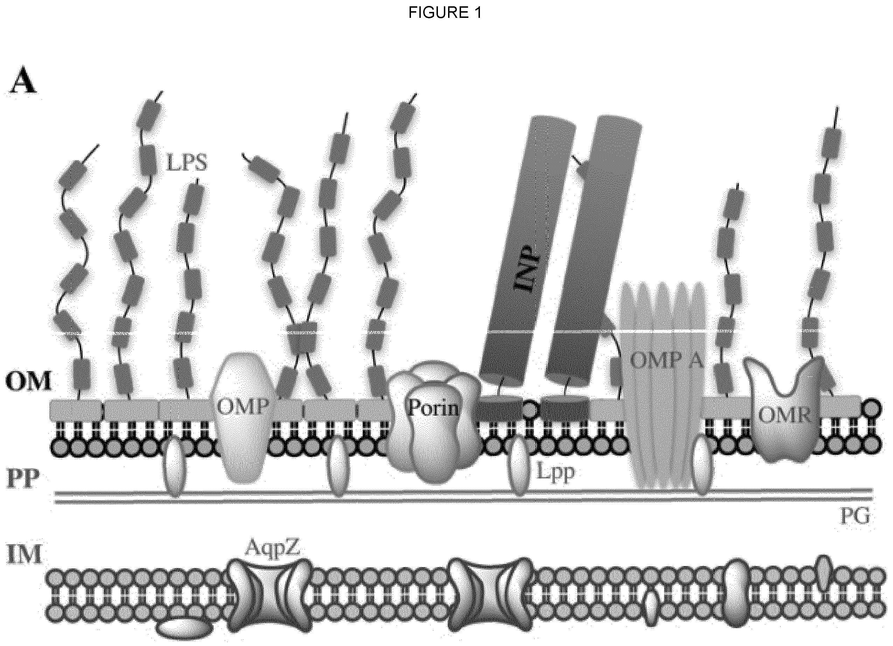

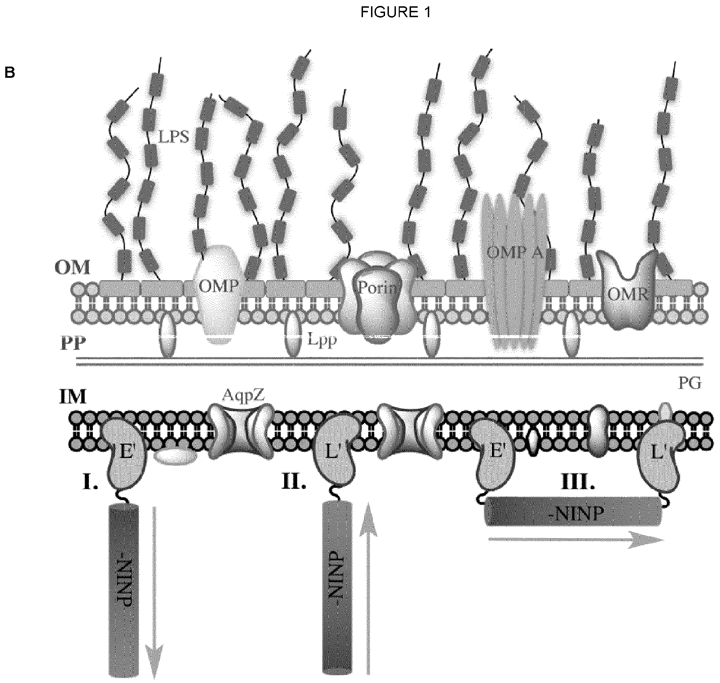

Figure 1A shows INPs located on BGs outer-membrane (OM). Figure 1B shows inner membrane targeting systems anchoring N-terminal truncated INP (-NINP). Figure 1B illustrates -NINP anchoring modes within the BG envelope, in particular -NINP fused to the amino-terminal sequence of the IM spanning polypeptide E' (I.), -NINP fused to the carboxy-terminal sequence of the IM spanning polypeptide L' (II.), or -NINP fused to both sequences (III.). OM: outer-membrane; PP: periplasm; IM: inner-membrane; LPS: lipopolysaccharide; OMP: outer-membrane protein; OMR: outer-membrane receptor; PG: peptidoglycan, Lpp: Braun's lipoprotein; AqpZ: aquaporin-Z; $\uparrow,\downarrow$: orientation of truncated InaZ fused to E', L' and E'-L' anchor sequences from N- to C-terminus.

Figure 2 shows plasmids constructed to anchor -NINP at the inner membrane of bacteria or of bacterial ghosts. Eivb: C-terminal fusion of gene E to an in-vivo biotinylation sequence

Figure 3 shows SFG spectra of an E'-NINP-BG layer for different temperatures at 20°C, 15°C, 10°C and 5°C. The SFG intensity increases with decreasing temperatures.

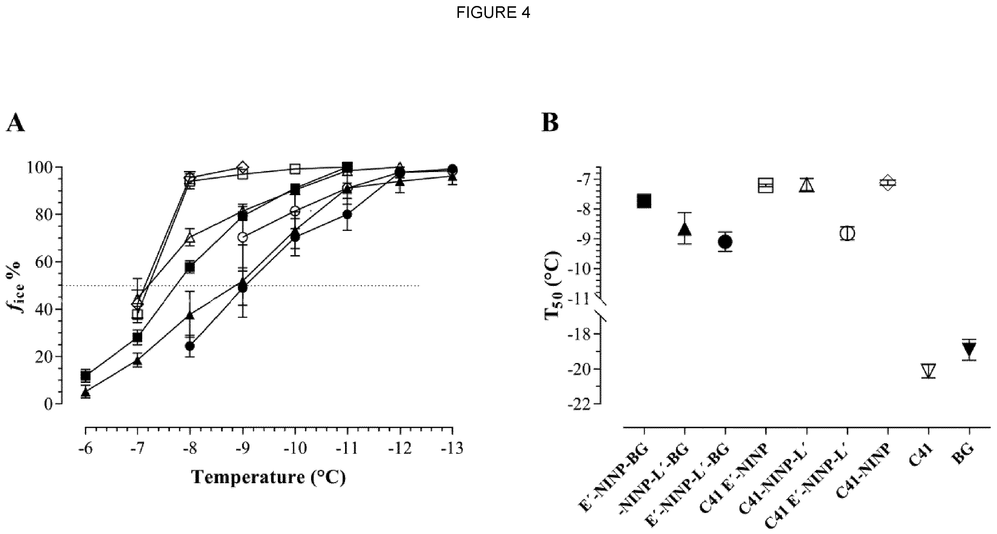

Figure 4 shows freezing spectra of E. coli C41 (open symbols) and their BG-derived versions (full symbols): C41 E'-NINP, E'-NINP-BG (square); C41 - NINP-L', -NINP-L'-BG (triangle); C41 E'-NINP-L', E'-NINP-L'-BG (circle) C41-NINP (diamond). Figure 4A shows a nucleation curve plotted as number fraction of frozen droplets in percent ($f_{ice}$%) at given temperatures. Figure 4B shows $T_{50}$(°C), i.e. the temperature where 50% of all droplets are frozen. Forty-five 10 $\mu$l droplets of each suspension containing $5 \times 10^8$ cells or were BGs ml$^{-1}$ were tested by droplet freezing. Also given are $T_{50}$ of living E. coli C41 (C41) and its derived BG form.

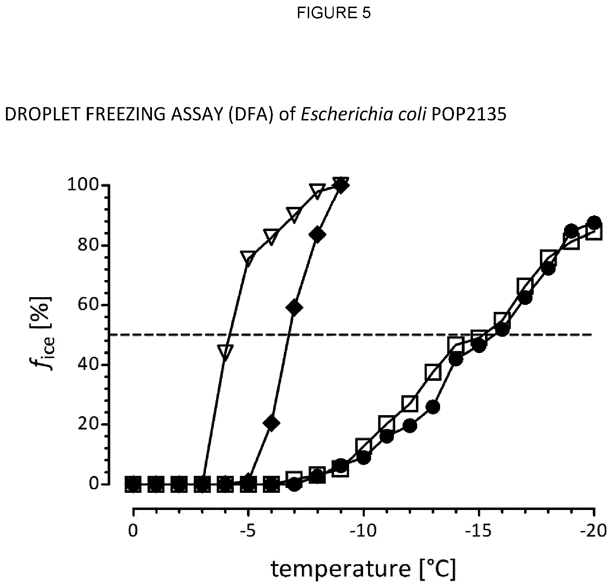

Figure 5 shows the results of a droplet freezing assay (DFA) for various preparations. In particular, Fig. 5A shows a droplet freezing assay (DFA) of E. coli POP2135 (•), of E. coli POP2135 carrying the ice nucleation protein InaZ expressed into the cytoplasm (E. coli POP2135 InaZ-cyto; (♦)) and of BGs of E. coli POP2135 carrying InaZ on the outer membrane (BGs of E. coli POP2135 InaZ-OM ($\nabla$)) illustrating ice nucleation curves plotted as number fraction of frozen droplets in percent ($f_{ice}$ %) at indicated temperatures. ROTISOLV® water ($\square$) was included as unspecific control. Each suspension contained $5 \times 10^8$ cells ml$^{-1}$. InaZ expressing strains were inactivated with 0.5% glutaraldehyde (GA). $T_{50}$ values are: E. coli POP2135 (•): -15.4°C; E. coli POP2135 InaZ-cyto (+): -6.73°C, BGs of E. coli POP2135 InaZ-OM ($\nabla$): - 3.81°C and Rotisolv® H$_2$O: -15.18°C

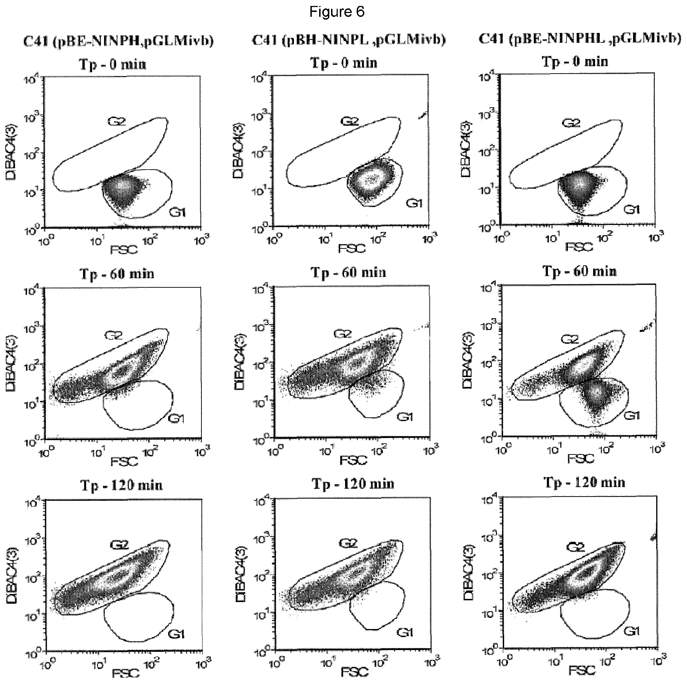

Figure 6 shows detection of -NINP fusion proteins in E. coli C41 and their derived BGs. Samples were taken before induction of E-mediated lysis (Tp-0min) (lane 1, 3, 5, 7) and after β-propiolactone treatment of BG samples (lane 2, 4, 6). Western blotting was performed with rabbit anti-H-NINP serum and anti-rabbit IgG horseradish peroxidase conjugated antibodies. Lane1: C41 (pBH-NINPL, pGLMivb), expressing -NINP-L; lane 2: -NINP-L-BG; lane 3: C41 (pBE-NINPH, pGLMivb), expressing E'-NINP; lane 4: E-NINP-BG; lane 5: C41 (pBE-NINPHL, pGLMivb), expressing E'-NINP-L'; lane 6: E'-NINP-L'-BG; Lane 7: C41 control (pBAD24, pGLMivb) harvested before lysis induction (OD$_{600}$ of 0.6); lane 8: BG form of C41 (pBAD24, pGLMivb); Lines indicate molecular size marker proteins in kilodaltons (kDa).

Figure 7 shows flow cytometry density dot plots during E-lysis process of E. *coli* C41 (pBE-NINPH, pGLMivb), C41 (pBH-NINPL, pGLMivb) and C41 (pBE-NINPHL, pGLMivb) strains. Online monitoring of BG production, starting at time point of lysis induction (Tp - 0min), after 60min of lysis (Tp - 60min) and end of lysis phase (Tp-120min). Dot plots illustrate fluorescence intensity with Dibac$_4$(3) versus - forward scatter (FSC); G1: living cells, G2: BGs.

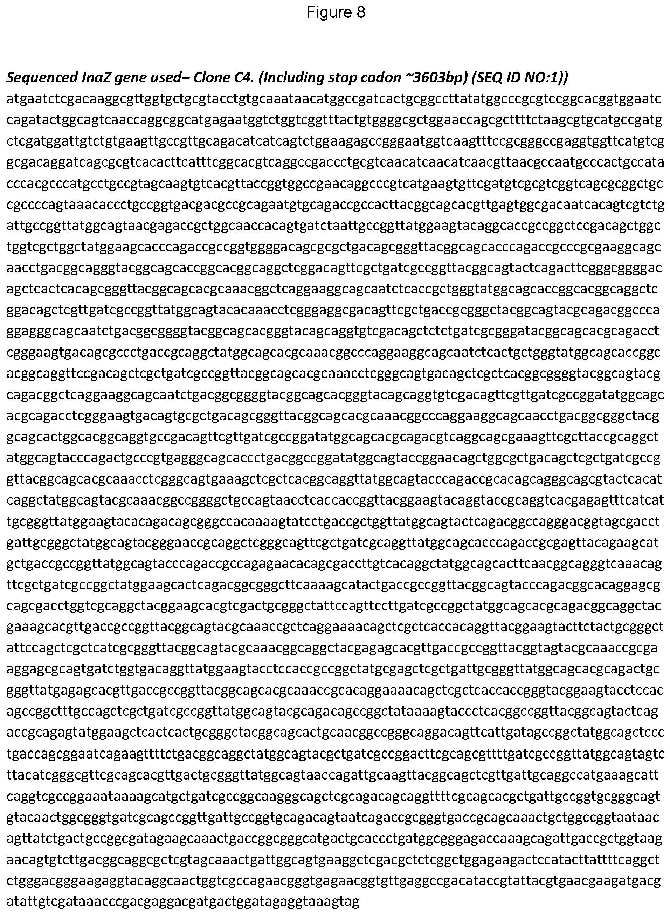

Figure 8 shows SEQ ID NO: 1 of the ice nucleation protein InaZ of Pseudomonas syringae.

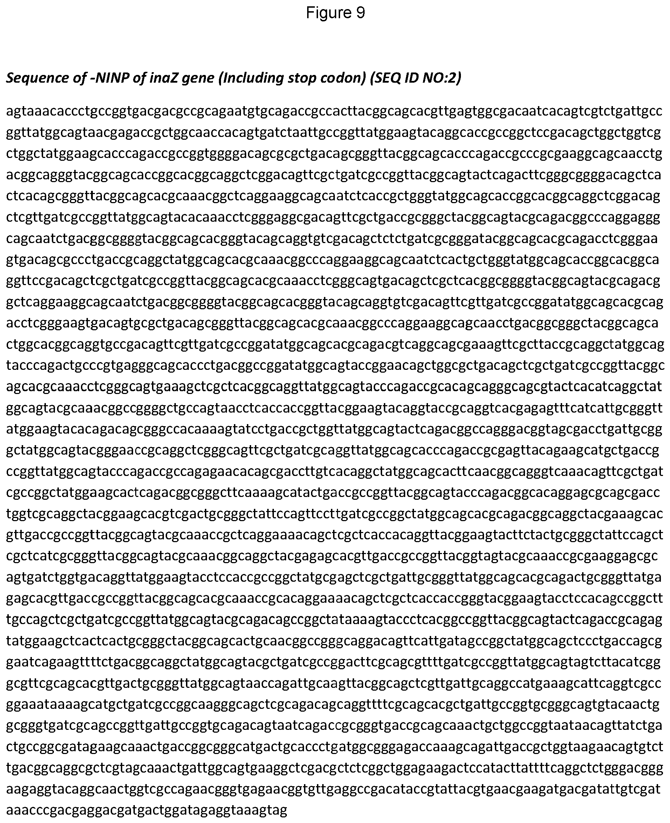

Figure 9 shows SEQ ID NO: 2, an N-truncated form of the ice nucleation protein InaZ of Pseudomonas syringae.

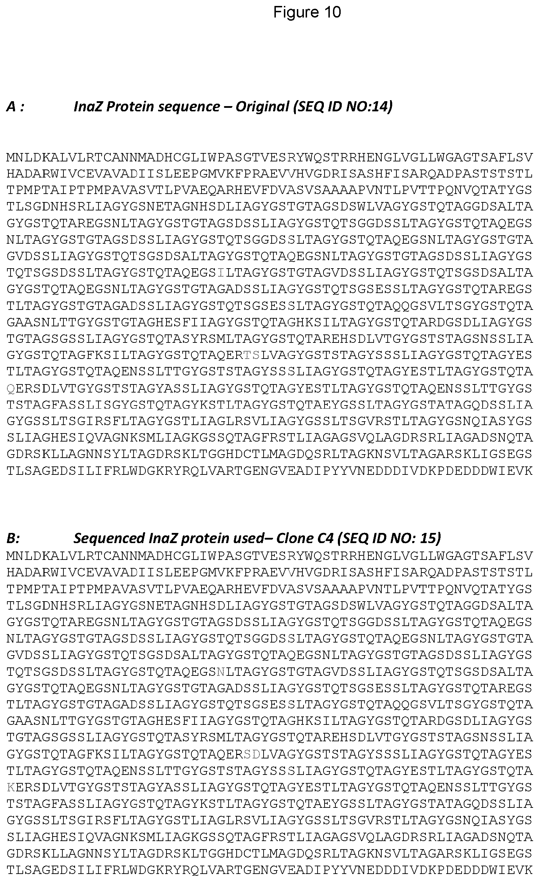

Figure 10 shows the protein sequence of the ice nucleation protein InaZ. Fig. 10A shows the original InaZ sequence, Fig. 10B the protein sequence of clone C4 having four amino acid replacements.

Example 1

Formation of Bacteria and Bacterial Ghosts

Bacterial ghosts (BGs) having a truncated form of InaZ localized to the inner membrane were produced. For the production of BGs exposing INPs to the luminal side of the inner membrane (IM), directional targeting of InaZ lacking 162aa of the 175aa-long N-terminal domain (-NINP) via N-terminal E'domain (E'-NINP), C-terminal L'domain (-NINP-L') or by fusing -NINP with both anchor peptides (E'-NINP-L') was used. -NINP is shown in SEQ ID NO: 2. In order to express different forms of IM anchored -NINP fusion proteins plasmids pBE-NINPH, pBH-NINPL and pBE-NINPHL were constructed.

Bacterial strains, plasmids and primers utilized are listed in Table 1.

| Strains, plasmids and primers | Description | Source or Reference |
|---|---|---|
| *Bacterial Strain* | | |
| *E. coli* C41 (DE3) | F - ompT hsdSB (rB- mB-) gal dcm (DE3). | Lucigen |
| *E. coli* K-12 5-α | F' proA⁺B⁺ lacIq Δ(lacZ)M15 zzf::Tn10 (Tet^R) / thuA2Δ(argF-lacZ)U169 phoA glnV44 Φ80Δ(lacZ)M15 gyrA96 recA1 relA1 endA1 thi-1 hsdR17. | NEB |
| *Plasmids* | | |
| pBAD24 | Bacterial expression vector containing the arabinose $P_{BAD}$ promotor system; restriction enzyme cloning; AmpR; ColE1 ori. | BIRD-C |
| pGLMivb | LacIq-$P_{TAc}$-Eivb; Gent^R. | |
| pEX-A2INP | $P_{LAC}$-inaZ, coding for InaZ, PUC Origin; Amp^R. | Eurofins Genomics |
| pBELK | $P_{BAD}$-E'-L'-cassette for inner-membrane anchoring of proteins, Kan^R. | BIRD-C |
| pBH-NINP | $P_{BAD}$-H-NINP; Amp^R. Coding for InaZ lacking N-terminal domain sequence with N-terminal His-tagged fusion. | |
| pBE-NINPHSLK | $P_{BAD}$-E'-NINP-His; Kan^R. Coding for E' -NINP-His fusion protein. | |
| pBE-NINPH | $P_{BAD}$-E'-NINP-His; Amp^R. Coding for E' -NINP-His fusion protein. | |
| pBH-NINPLK | $P_{BAD}$-His-NINP-L'; Kan^R. Coding for His -NINP-L' fusion protein. | |
| pBH-NINPL | $P_{BAD}$-His-NINP-L'; Amp^R. Coding for His -NINP-L' fusion protein. | |
| pBE-NINPHLK | $P_{BAD}$-E'-NINPhis-L'; Kan^R. Coding for E'-NINPHis-L' fusion gene. | |
| pBE-NINPHLK | $P_{BAD}$-E'-NINPhis-L'; Amp^R. Coding for E'-NINPHis-L' fusion gene. | |
| *Primers* | *Sequence (5'→ 3')* | *Restriction site* |
| P1: -N_INP-fwd | GTACGCTCTAGAAGTAAACACCCTGCCGGT | *XbaI* |
| P2: INP-His-rev | AAAAAACTGCAGTTATTAATGGTGATGGTGATGGTGAGAGCCGGATCCCTTTACCTCTATCCAGTCATC | *PstI* |
| P3: E'-fwd | GTACCGGAATTCTTTATGGTACGCTGGACT | *EcoRI* |
| P4: His-tag-rev | GACCCAAGCTTGCAGTTATTAATGGTGATGG | *HindIII* |

(continued)

| Primers | Sequence (5'→ 3') | Restriction site |
|---|---|---|
| P5: His-NINP-fwd | GTACCG<u>GAATTC</u>ACTACTC*ATGCACCATCACCATCACCA*TGGATCCGGC<u>T</u>CTGTAAACACCCTGCCGGT | *EcoRI* |
| P6: INP-rev | AAAAAA<u>CTGCAG</u>ACTTTACCTCTATCCAGTCATC | *PstI* |
| P7: His-tag-fwd | GTACCGGAATTCCATGCACCATCACCATC | *EcoRI* |
| P8: L'-rev | GTACGC<u>TCTAGA</u>CTTTGTGAGCAATTCGTC | *XbaI* |
| P9:INP-His1-rev | AAAAAA<u>CTGCAGA</u>*ATGGTGATGGTGATGGTG*AGAGCCGGATCCCTTTACCTCTATCCAGTCATC | *PstI* |
| P10: L'1-rev | AACATG<u>CCATGG</u>CTTTGTGAGCAATTCGTC | *NcoI* |

The primer restriction sites are underlined and 6x His-tag sequence is highlighted in italic.

The E. *coli* strain K-12 5-$\alpha$ has been used for routine cloning and E. *coli* C41 (DE3) (Lucigen) for BG production. Plasmid pBAD24, E-lysis plasmid pGLMivb and plasmid pBELK were obtained from BIRD-C plasmid collection. Plasmid pBELK contains an E'- L'- anchoring cassette derived from pKSEL5-2 under control of the arabinose inducible $P_{BAD}$ promoter. Plasmid pEX-A2INP (Eurofins Genomics) harbors a chemically synthesized 3603 bp *inaZ* gene encoding INP of *P. syringae* S203. The sequence thereof is given as SEQ ID NO: 1. In lysis plasmid pGLMivb expression of the lysis gene *E* is under control of $P_{TAC}$ promoter and translational fused to an in vivo biotinylation sequence. Expression vector pBH-NINP, coding for a N-terminal His-tagged truncated INP lacking 486 bp of the 525 bp long N-terminal domain (H-NINP) has been described in J. Kassmannhuber et al., Functional Display of Ice Nucleation Protein InaZ on the surface of Bacterial Ghosts, Bioengineering 2017.

In order to construct a fusion between INP and the E'-anchor first a truncated INP lacking N-terminal domain (-NINP) (lacking first 485 nt) was generated. A 3171 bp fragment absent of INP N-domain sequence was produced by PCR amplification using plasmid pEX-A2INP as template and primers P1 and P2 to introduce *XbaI*- and *PstI*- restriction sites at the termini and a 6x-His tag coding sequence at 3'-end with a terminal coding sequence. The amplified PCR-product coding for -NINP-His was cloned into the corresponding sites of pBELK resulting in plasmid pBE-NINPHSLK. The 3338 bp translational fused *E'-NINP-His* PCR-fragment was amplified using pBE-NINPHSLK as template and primers P3 and P4 containing *EcoRI* and *HindIII* at terminal restriction sites. The fragment was cloned into the equivalent sites of pBAD24 to construct the E'-NINP-His anchor fusion protein expression-vector pBE-NINPH under transcriptional control of the arabinose-inducible expression system.

Using pEX-A2INP as template a 3171 bp PCR-fragment, encoding the His-NINP protein without termination codon, was obtained by PCR amplification. Primers P5 and P6 were used to introduce a 6x-His tag coding sequence at 5'-end and *EcoRI*- *PstI*- restriction sites at the terminal ends. In frame fusion of the L'- anchor and His-NINP sequence was generated by cloning the fragment into the equivalent sites of pBELK resulting in pBH-NINPLK. By PCR using pBH-NINPLK as template and primers P7 and P8 to introduce restriction sites *EcoRI* and *XbaI* at the termini a 3366 bp PCR-fragment was obtained encoding the anchor fusion protein His-NINP-L'. The fragment was cloned into the corresponding sites of pBAD24 resulting in pBH-NINPL.

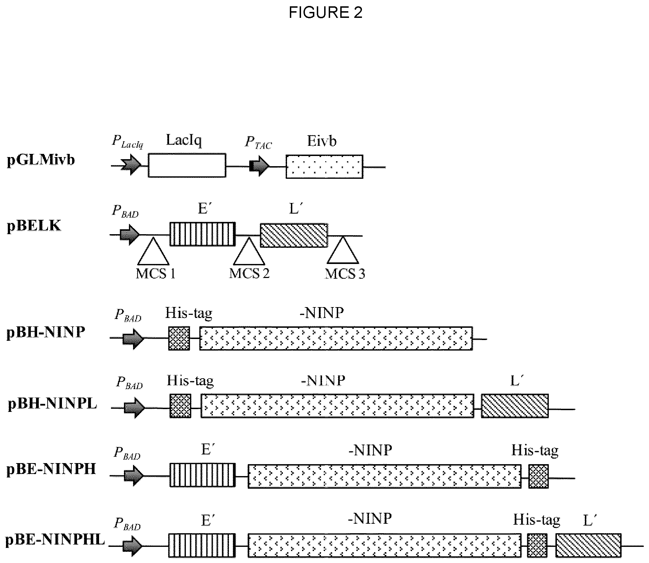

A 3165 bp PCR-fragment was generated by using P1 and P9 primers and pEX-A2INP as template to obtain the -NINP-His gene without a terminal coding sequence and 5'*XbaI* and 3'*PstI* restriction sites. The fragment was cloned into the *XbaI/PstI* sites of pBELK resulting in pBE-NINPHLK carrying the E'-*NINPH* - L' fusion gene, which translational fuses the -NINP-His sequence to the amino-terminal E' sequence and the carboxy-terminal L' sequence. The *E' -NINP* - L' gene was amplified by using primers P3 and P10 to introduce *EcoRI* and *NcoI* restriction sites at the termini. The 3528 bp fragment was cloned into the corresponding sites of pBAD24 resulting in pBE-NINPHL. Figure 2 illustrates the plasmids used and constructed for production of BGs carrying cytoplasmic anchored INPs facing the BG luminal site.

Bacterial cultures were grown in animal protein-free, vegetable variant of Luria-Bertani (LBv: 10.0 g/l soy peptone (Car Roth) 5.0 g/l yeast extract (Carl Roth) and 5.0 g/l NaCl) and supplemented with appropriate antibiotics, ampicillin (100$\mu$g / ml), gentamycin (20$\mu$g / ml) and kanamycin (50$\mu$g / ml) at 37° or 23°C. To induce expression of the anchor fusion gene which is under the control of $P_{BAD}$ promoter the E. *coli* C41 cells carrying plasmid (pBE-NINPH, pBH-NINPL, or pBE-NINPHL) were grown in LBv supplemented with 0.2 % L-arabinose at 23°C. In plasmid pGLMivb the expression of lysis gene

E is under the control of synthetic $P_{TAC}$ promotor. The bacterial lysis was induced with 0.5 mM isopropyl-D-1-thiogalactopyranoside (IPTG). Gene E-mediated lysis of bacteria was induced when cells reached an optical density at 600 nm ($OD_{600}$) of 0.6, and was extended for duration of 120min. At the end of the E-lysis procedure the BGs were harvested and washed four times with 1x Vol. of sterile de-ionized water ($dH_2O$) by centrifugation and finally resuspended in 1x Vol. of sterile $dH_2O$. For inactivation of any surviving E-lysis escape mutants from BG production representing a minor fraction of about 0.1 % - 0.3 % the washed BGs harvest was treated with 0.17 % (v/v) of the DNA-alkylating agent β-propiolactone (BPL, 98.5 %, Ferak) and kept for 120min at 23°C with slow agitation. After inactivation process, the fully inactivated cell broth consisting of BGs and inactivated surviving cells were washed twice with 1x Vol. of sterile $dH_2O$ and once with ROTISOLV® water (Carl Roth) and resuspended in 1/10x Vol. ROTISOLV® water. The suspensions of E. *coli* C41 cells and BGs carrying cytoplasmic membrane anchored ice nucleation protein were adjusted to a concentration of 5 x $10^8$ cells ml$^{-1}$ determined by flow cytometry (FCM) in ROTISOLV® water.

## Example 2

### Determination of colony forming units (cfu)

For cfu determination appropriate dilutions of samples (0.85 % (w/v) NaCl Solution) were plated on Plate Count Agar (PCA) (Carl Roth) using a WASP spiral plater (Don Whitley Scientific). 50 μl samples were plated on PCA plates as triplicates. The plates were incubated at 35°C over night and the next day the cfu was analyzed by a ProtoCOL SR 92000 colony counter (Synoptics Ltd); Lysis efficiency (LE) is defined as ratio of BGs to total cell counts and can be calculated as following equation:

$$LE = \left(1 - \frac{cfu_{(t)}}{cfu_{(t_0)}}\right) \times 100\% \tag{1}$$

where $t_0$ is the time point of lysis induction (LI) and t any time after LI.

Lysis efficiency (LE) for E. coli C41 carrying plasmids (pBE-NINPH and pGLMivb) amounts to 99.9%; for E. coli C41 carrying plasmid (pBH-NINPL and pGLMivb) a LE of 99.8% and for E. coli C41 harboring plasmids (pBE-NINPHL and pGLMivb) a LE of 99.7% was achieved.

### E-lysis **monitoring**

The BG production was monitored by light-microscopy (Leica DM R microscope, Leica Microsystems), by measuring the optical density at 600 nm ($OD_{600}$) and fluorescence-based flow cytometry (FCM). Briefly, flow cytometry was performed using a CyFlow® SL flow cytometer (Partec) and the membrane potential-sensitive dye DiBAC4(3) was well as the phospholipid membranes staining RH414 (both from AnaSpec) were used for fluorescent labeling, Dye RH414 was used for discriminating non-cellular background and DiBAC for the evaluation of cell-viability. Data were analyzed using FloMax V 2.52 (CyFlow SL; Quantum Analysis) illustrating forward scatter (FSC) against DiBAC fluorescence signal (FL1, DiBAC) and presented as 2D density dot plots as shown in Fig. 6.

The fluorescent dye RH414 staining phospholipid membranes enables discrimination of non-cellular background and DiBAC4(3) penetrating depolarized cell membranes binding to intracellular proteins or membrane compartments signaling changes in the membrane potential were used. A complete switch of DiBAC-negative cells with high scatter signal (G1), to DiBAC-positive cells with a diminished scatter signal (G2) marks the completion of protein E-mediated lysis process (Fig. 6).

In order to inactivate E-lysis escape mutants, the cultures were further incubated at 23°C and treated with 0.17 % (v/v) β-propiolactone for another 120 min. In the final preparation of E. *coli* BGs carrying either E'-NINP (E'-NINP-BG), -NINP-L'(-NINP-L'-BG) or E'-NINP-L' (E'-NINP-L'-BG) no viable cells were detected.

### Western blot analyses

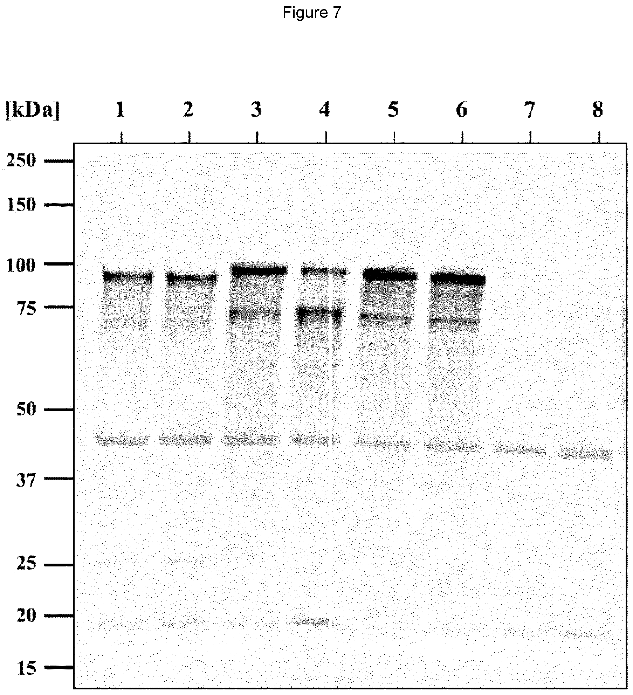

Pellets of 5 x $10^{-8}$ cells or BGs, respectively per ml were boiled in SDS gel-loading buffer (1x) for 5min and separated on Bolt™ 4-12 % Bis-Tris Plus gel by using a XCell SureLock™ Mini-Cell electrophoresis system (Thermo Fisher Scientific). By using XCell II™ Blot Module (Thermo Fisher Scientific) the electrophoretically separated proteins were then transferred to nitrocellulose membrane (GE Healthcare) with transfer buffer (25 mM Tris, 192mM glycine, 20 % methanol). The membrane was placed in TBST (20 mM Tris-HCl, pH7.5, 100 mM NaCl, 0.05 % Tween-20) with 5 % milk powder (Carl Roth) overnight at 4°C. Polyclonal α-H-NINP serum from rabbit was used to detect recombinant INP. Immunodetection was performed using α-H-NINP serum followed by α-rabbit IgG-HRP (GE Healthcare). Detection was performed using Amersham ECL Western blot detection kit (GE Healthcare) and developed with ChemiDoc™ XRS (Bio-Rad). The results are shown in Fig. 7.

The predicted molecular mass ($M_r$) of the fusion protein E'-NINP is 109.2 kDa, $M_r$ of -NINP-L is 110.3 kDa and of E'-NINP-L' 116.9 kDa. The full length -NINP forms were detected around their specific $M_r$. Apart from the unspecific binding of the used polyclonal antibodies with proteins derived from E. *coli,* the lower migrated bands around 75 kDa most probably represent degradation products of the above mentioned INPs.

## Example 3

### Measurement of ice nucleation activity

Ice nucleation activities of E. coli C41 constructs and BG-derived versions carrying either E'-INP, -NINP-L' or E'-NINP-L' were determined by a droplet-freezing assay. Additionally, full E. coli C41 cells carrying pBH-NINP encoding a cytoplasmic N-terminal His-tagged truncated INP lacking 162 aa of N-terminal outer-membrane binding domain (C41-NINP) were tested for their ice-nucleating activity.

Out of each suspension to be tested, forty-five 10 $\mu$l droplets of each tenfold dilutions series (ranging from 5 x $10^8$ cells $ml^{-1}$ to 5 x $10^4$ cells $ml^{-1}$) were tested for active ice nuclei inside the droplet at a given temperature. The droplets were distributed on a sterile aluminum plate coated with a hydrophobic film and surrounded by styrofoam and covered by a plexiglas plate for isolation. The temperature of the working plate was decreased by two in series circuited two-stage Peltier elements of the type TEC2-127-63-04. The surface temperature of the plate was measured by a small precision temperature sensor TS-NTC-103A (B+B Thermo-Technik). Ice nucleation activity was tested from -2 to -13°C at a constant rate of 1°C decrements. After a 30 sec dwell time at each temperature the Plexiglas plate was removed and the number of frozen droplets was recorded. The results are shown in Fig. 4.

## Example 4

### Determination of ice nucleation activity

Forty-five (of 10 $\mu$l volume) drops containing a known number of BGs or bacterial cell suspension was allowed to cool to a fixed temperature as in Example 3 and the number of frozen droplets were counted. This measurement was repeated for each and every series of 10-fold dilutions to obtain statistically significant values The different samples were compared by their median freezing temperature ($T_{50}$), which represents the temperature where 50 % of all droplets are frozen. The $T_{50}$ was calculated with the equation,

$$T_{50} = \frac{T_1 + (T_2 - T_1)(2^{-1}n - F_1)}{(F_2 - F_1)} \tag{2}$$

where, F1 and F2 are the number of frozen droplets at temperature T1 and adjacent temperature T2, and are just below and above 50 % of the total number of tested drops (n).

The cumulative number N(T), of ice nuclei $ml^{-1}$ active at a given temperature was calculated by

$$N(T) = -\ln(f) \times \frac{10^D}{V} \tag{3}$$

where, f = fraction of unfrozen droplets at temperature T, V = volume of each droplet used (10 $\mu$l), D = the number of 1:10 serial dilutions of the original suspension. N(T) was normalized for the number of cells present in each suspension to obtain the nucleation frequency (NF) per cell by dividing ice nuclei -ml through cell density (cell-ml)

The results are shown in Figs. 4A and 4B.

The first frozen droplets of E. coli C41 cells without INP (median freezing temperature, $T_{50}$, of -20.1°C) and E. coli C41 BGs without INP ($T_{50}$ of -18.9°C) were detected at -14°C.

BGs carrying E'-NINP fusion protein (E'-NINP-BG) showed a $T_{50}$ value at - 7.7°C, $T_{50}$ for BGs carrying -NINP-L' (-NINP-L'-BG) was determined at - 8.7°C and for BGs with E'-NINP-L' anchored (E'-NINP-L'-BG) a $T_{50}$ at -9°C was recorded.

For C41-NINP a $T_{50}$ of -7.1°C was determined, for C41 E'-NINP, a $T_{50}$ of - 7.2°C, for C41-NINP-L' a $T_{50}$ of -7.2°C and for C41 E'-NINP-L' a $T_{50}$ of - 8.8°C.

C41-NINP carries cytoplasmic N-terminal truncated INP while in C41 E'-NINP, C41-NINP-L' and C41 E'-NINP-L' the ice nuclein proteins are anchored at the inner membrane.

## Example 5

**Sum frequency generation spectroscopy**

To test the impact of INPs in BG on the water structure sum frequency generation (SFG) vibrational spectroscopy was used. SFG uses frequency mixing of infrared and visible laser pulses to probe the molecular structure of interfaces.

The SFG setup is based on a Ti:sapphire fs-laser oscillator (MaiTai, Spectra-Physics). A regenerative amplifier (SpitFire Ace, Spectra-Physics) pumped by a Nd:YLF laser (EMPower, Spectra-Physics) is used to generate a 9,5 mJ pulse at 800 nm with a 40 fs duration at a repetition rate of 1 kHz. 1.5 mJ of the output energy is used to pump a commercial optical parametric amplifier (TOPAS-C, Spectra-Physics). The signal and idler pulses of the parametric amplifier are mixed in a silver gallium disulfide ($AgGaS_2$) difference frequency generation crystal, resulting in 3 $\mu$J IR pulses, centered at 2500 $cm^{-1}$ with a full width at half maximum (FWHM) of ~800 $cm^{-1}$. The narrowband visible (VIS) up-conversion pulses (25 $\mu$J, FWHM-15 $cm^{-1}$) are obtained by passing 800 nm pulses (1 mJ pulse energy) through a Fabry-Perot etalon (SLS Optics Ltd). The visible and IR beams are spatially and temporally overlapped on the sample surface with incident angles of 45° and 50°, respectively, with respect to the surface normal. The desired ssp (s-polarized SFG, s-polarized VIS, p-polarized IR) polarization is obtained using polarizer and half wave plates in combination. The VIS and IR beams are focused on the sample with 20cm and 5cm focal length plano-convex lenses respectively. The sum-frequency signal is collected in reflection geometry and collimated by a 20 cm focal length lens before passing through a short wave pass filter to remove the residual visible light. The polarization of the SFG light is controlled by polarization optics before it is guided to a spectrograph (Acton Instruments) and detected with an electron-multiplied charge-coupled device (EMCCD) camera (Newton; Andor Technologies). All SFG spectra were recorded under ssp polarization conditions. The spectra where recorded over 5 minutes. The SFG sample area and the IR beam path were flushed with nitrogen to avoid spectral artifacts from water vapor. All the SFG spectra were normalized using reference spectra obtained from z-cut quartz.

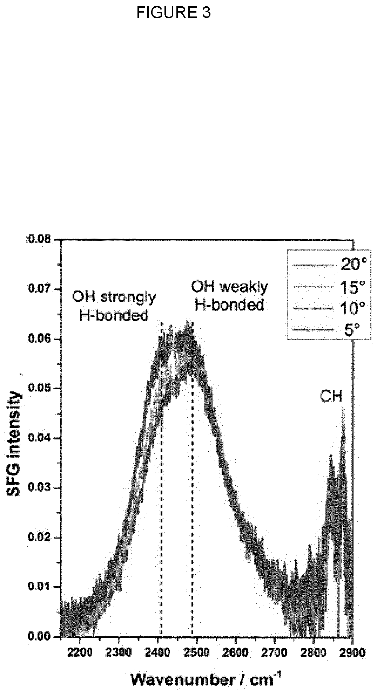

The results are shown in Fig. 3.

The interaction of E'-NINP-BG with water was probed with SFG for BGs assembled at the air-water interface. SFG spectra of the BG monolayers were collected in the ssp (s-polarized SFG, s-polarized visible, p-polarized infrared) polarization combination. The spectra show C-H resonances between 2900 $cm^{-1}$ and 2800 $cm^{-1}$. These resonances are related to methylene units within carbohydrates, lipids, protein side chains and other organic molecules present in the BGs. Strong modes in the O-D stretching range between 2200 and 2700 $cm^{-1}$ show the presence of ordered water molecules at the bacterial membrane surfaces. Disordered water near the membrane is not detected with SFG. The room temperature spectrum (blue trace) shows a broad peak with components related to weakly and strongly hydrogen-bonded water molecules. Spectra collected at 15°C, 10°C, and 5°C showed an increase of the SFG water signal, indicating that more ordered water is present at the surface. The data clearly indicate that INPs within the BGs orient water within their hydration shell when cooled to appropriate temperatures.

**Example 6**

**Surface Tension Experimental Details**

Surface tension has been measured using a Langmuir tensiometer (Kibron, Finland). E'-NINP-BG have been prepared in $D_2O$. The temperature-controlled trough was thoroughly rinsed with acetone, ethanol and milli-Q water, and dried under nitrogen stream prior to measurements. The tensiometer was calibrated using pure $D_2O$ at room temperature (20 °C).

**Example 7**

**Inactivation with glutaraldehyde (GA)**

E. coli POP2135 bacteria having the ice nucleation protein InaZ expressed into the cytoplasm (E. coli POP2135 InaZ-cyto) were prepared.

Further, bacterial ghosts from E. coli POP2135 carrying InaZ on the outer membrane (E. coli POP2135 InaZ-OM) were prepared.

Cell suspensions of E. coli POP2135 InaZ-cyto as well as of E. coli POP2135 InaZ-OM were washed with 0.3% sodium bicarbonate. Thereafter, glutaraldehyde (GA) was added to the suspensions and inactivation/fixation was performed at room temperature (20°C to 23°C) at GA concentrations of 0.02% (v/v), 0.05% (v/v), 0.1% (v/v), 0.2% (v/v), 0.5% (v/v), 1% (v/v) and 2% (v/v). Cell suspensions containing 1 x $10^{10}$ bacteria or ghosts, respectively, per ml were incubated for 15 minutes up to overnight. Cell suspensions containing 5 x $10^{10}$ bacteria or ghosts, respectively, per ml were inactivated for 15 minutes up to overnight.

A droplet-freezing assay (DFA) was carried out with the obtained inactivated and fixed bacterial preparations.

The results are shown in Fig. 5. As can be seen therefrom, the $T_{50}$ value for E. coli POP2135 InaZ-cyto is -6.73°C

and the $T_{50}$ value of BG E. coli POP2135 InaZ-OM is -3.81°C. This is a clear increase in the freezing temperature as compared to the unspecific control ROTISOLV $H_2O$, which showed a $T_{50}$ value of -15.18°C as well as compared to empty E. coli POP2135 bacteria, which showed a $T_{50}$ value of -15.4°C.

**Claims**

1. Bacterial preparation comprising

inactivated bacteria, bacterial ghosts (BGs) or fragments thereof which carry an ice nucleation protein (INP) being the ice nucleation protein Z (InaZ) of Pseudomonas syringae and which are fixed with the fixation agent glutaraldehyde, wherein the bacterial preparation
comprises inactivated bacteria, wherein the ice nucleation protein (INP) is within the cytoplasm and/or
comprises bacterial ghosts (BGs) from Gram-negative bacteria which carry the ice nucleation protein (INP) anchored at the outer membrane (OM); and
wherein the ice nucleation protein (INP) being the ice nucleation protein Z (InaZ) of Pseudomonas syringae is heterologous to the inactivated bacteria or bacterial ghosts (BGs).

2. Bacterial preparation according to claim 1,
**characterized in that**
it comprises an ice nucleation protein (INP) being the ice nucleation protein Z (InaZ) of Pseudomonas syringae lacking a transport sequence for localization in the outer membrane.

3. Bacterial preparation according to any one of claims 1 to 2,
wherein the inactivated bacteria are E. coli bacteria and/or the bacterial ghosts are E. coli bacterial ghosts or fragments thereof.

4. Use of a bacterial preparation according to any one of claims 1 to 3 for ice nucleation, for the production of snow, for the production of rain, for cloud seeding, weather modification, as cloud condensation nuclei (CNN), for artificial cloud creation, for ice nucleation in the atmosphere, for hail suppression or for fog dispersal.

**Patentansprüche**

1. Bakterienpräparat, umfassend inaktivierte Bakterien, Bakterienghosts (BGs) oder Fragmente davon, die ein Eisnukleationsprotein (INP) tragen, das das Eisnukleationsprotein Z (InaZ) von Pseudomonas syringae ist, und die mit dem Fixiermittel Glutaraldehyd fixiert sind, wobei das Bakterienpräparat inaktivierte Bakterien umfasst, wobei sich das Eisnukleationsprotein (INP) im Zytoplasma befindet und/oder
Bakterienghosts (BGs) von gramnegativen Bakterien umfasst, die das Eisnukleationsprotein (INP) an der äußeren Membran (OM) verankert tragen; und wobei das Eisnukleationsprotein (INP), das das Eisnukleationsprotein Z (InaZ) von Pseudomonas syringae ist, zu den inaktivierten Bakterien oder Bakterienghosts (BGs) heterolog ist.

2. Bakterienpräparat nach Anspruch 1,
**dadurch gekennzeichnet, dass**
es ein Eisnukleationsprotein (INP) umfasst, bei dem es sich um das Eisnukleationsprotein Z (InaZ) von Pseudomonas syringae handelt, dem eine Transportsequenz für die Lokalisierung in der äußeren Membran fehlt.

3. Bakterienpräparat nach einem der Ansprüche 1 bis 2,
wobei die inaktivierten Bakterien E. coli-Bakterien und/oder die Bakterienghosts E. coli-Bakterienghosts oder Fragmente davon sind.

4. Verwendung einer Bakterienpräparation gemäß einem der Ansprüche 1 bis 3 zur Eisnukleation, zur Erzeugung von Schnee, zur Erzeugung von Regen, zur Wolkenbildung, zur Wetterveränderung, als Wolkenkondensationskerne (CNN), zur künstlichen Wolkenbildung, zur Eisnukleation in der Atmosphäre, zur Hagelunterdrückung oder zur Nebelauflösung.

**Revendications**

1. Préparation bactérienne comprenant

   des bactéries inactivées, des fantômes bactériens (BG) ou des fragments de ceux-ci qui portent une protéine de nucléation de glace (INP) qui est la protéine de nucléation de glace Z (InaZ) de Pseudomonas syringae et qui sont fixés avec l'agent de fixation glutaraldéhyde, dans laquelle la préparation bactérienne

   comprend des bactéries inactivées, dans laquelle la protéine de nucléation de glace (INP) se trouve à l'intérieur du cytoplasme et/ou

   comprend des fantômes bactériens (BG) provenant de bactéries Gram-négatives qui portent la protéine de nucléation de glace (INP) ancrée à la membrane externe (OM) ; et

   dans laquelle la protéine de nucléation de glace (INP) qui est la protéine de nucléation de glace Z (InaZ) de Pseudomonas syringae est hétérologue aux bactéries inactivées ou aux fantômes bactériens (BG).

2. Préparation bactérienne selon la revendication 1,
   **caractérisée en ce que**
   elle comprend une protéine de nucléation de glace (INP) qui est la protéine de nucléation de glace Z (InaZ) de Pseudomonas syringae dépourvue d'une séquence de transport pour la localisation dans la membrane externe.

3. Préparation bactérienne selon l'une quelconque des revendications 1 à 2, dans laquelle les bactéries inactivées sont des bactéries E. coli et/ou les fantômes bactériens sont des fantômes bactériens E. coli ou des fragments de ceux-ci.

4. Utilisation d'une préparation bactérienne selon l'une quelconque des revendications 1 à 3 pour la nucléation de glace, pour la production de neige, pour la production de pluie, pour l'ensemencement de nuage, la modification météorologique, sous forme de noyaux de condensation de nuages (CNN), pour la création de nuages artificiels, pour la nucléation de glace dans l'atmosphère, pour l'élimination de la grêle ou pour la dispersion du brouillard.

FIGURE 1

FIGURE 1

FIGURE 2

FIGURE 3

FIGURE 4

FIGURE 5

DROPLET FREEZING ASSAY (DFA) of *Escherichia coli* POP2135

Figure 6

Figure 7

Figure 8

*Sequenced InaZ gene used– Clone C4. (Including stop codon ~3603bp) (SEQ ID NO:1))*

atgaatctcgacaaggcgttggtgctgcgtacctgtgcaaataacatggccgatcactgcggccttatatggcccgcgtccggcacggtggaatc
cagatactggcagtcaaccaggcggcatgagaatggtctggtcggtttactgtggggcgctggaaccagcgcttttctaagcgtgcatgccgatg
ctcgatggattgtctgtgaagttgccgttgcagacatcatcagtctggaagagccgggaatggtcaagtttccgcgggccgaggtggttcatgtcg
gcgacaggatcagcgcgtcacacttcatttcggcacgtcaggccgaccctgcgtcaacatcaacatcaacgttaacgccaatgcccactgccata
cccacgcccatgcctgccgtagcaagtgtcacgttaccggtggccgaacaggcccgtcatgaagtgttcgatgtcgcgtcggtcagcgcggctgc
cgcccagtaaacaccctgccggtgacgacgccgcagaatgtgcagaccgccacttacggcagcacgttgagtggcgacaatcacagtcgtctg
attgccggttatggcagtaacgagaccgctggcaaccacagtgatctaattgccggttatggaagtacaggcaccgccggctccgacagctggc
tggtcgctggctatggaagcacccagaccgccggtggggacagcgcgctgacagcgggttacggcagcacccagaccgcccgcgaaggcagc
aacctgacggcagggtacggcagcaccggcacggcaggctcggacagttcgctgatcgccggttacggcagtactcagacttcgggcggggac
agctcactcacagcgggttacggcagcacgcaaacggctcaggaaggcagcaatctcaccgctgggtatggcagcaccggcacggcaggctc
ggacagctcgttgatcgccggttatggcagtacacaaacctcgggaggcgacagttcgctgaccgcgggctacggcagtacgcagacggccca
ggagggcagcaatctgacggcggggtacggcagcacgggtacagcaggtgtcgacagctctctgatcgcgggatacggcagcacgcagacct
cgggaagtgacagcgccctgaccgcaggctatggcagcacgcaaacggcccaggaaggcagcaatctcactgctgggtatggcagcaccggc
acggcaggttccgacagctcgctgatcgccggttacggcagcacgcaaacctcgggcagtgacagctcgctcacggcgggggtacggcagtacg
cagacggctcaggaaggcagcaatctgacggcggggtacggcagcacgggtacagcaggtgtcgacagttcgttgatcgccggatatggcagc
acgcagacctcgggaagtgacagtgcgctgacagcgggttacggcagcacgcaaacggcccaggaaggcagcaacctgacggcgggctacg
gcagcactggcacggcaggtgccgacagttcgttgatcgccggatatggcagcacgcagacgtcaggcagcgaaagttcgcttaccgcaggct
atggcagtacccagactgcccgtgagggcagcaccctgacggccggatatggcagtaccggaacagctggcgctgacagctcgctgatcgccg
gttacggcagcacgcaaacctcgggcagtgaaagctcgctcacggcaggttatggcagtacccagaccgcacagcagggcagcgtactcacat
caggctatggcagtacgcaaacggccgggggctgccagtaacctcaccaccggttacggaagtacaggtaccgcaggtcacgagagtttcatcat
tgcgggttatggaagtacacagacagcgggccacaaaaagtatcctgaccgctggttatggcagtactcagacggccagggacggtagcgacct
gattgcgggctatggcagtacgggaaccgcaggctcgggcagttcgctgatcgcaggttatggcagcacccagaccgcgagttacagaagcat
gctgaccgccggttatggcagtacccagaccgccagagaacacagcgaccttgtcacaggctatggcagcacttcaacggcagggtcaaacag
ttcgctgatcgccggctatggaagcactcagacggcgggcttcaaaaagcatactgaccgccggttacggcagtacccagacggcacaggagcg
cagcgacctggtcgcaggctacggaagcacgtcgactgcgggctattccagttccttgatcgccggctatggcagcacgcagacggcaggctac
gaaagcacgttgaccgccggttacggcagtacgcaaaccgctcaggaaaacagctcgctcaccacaggttacggaagtacttctactgcgggct
attccagctcgctcatcgcgggttacggcagtacgcaaacggcaggctacgagagcacgttgaccgccggttacggtagtacgcaaaccgcga
aggagcgcagtgatctggtgacaggttatggaagtacctccaccgccggctatgcgagctcgctgattgcgggttatggcagcacgcagactgc
gggttatgagagcacgttgaccgccggttacggcagcacgcaaaccgcacaggaaaacagctcgctcaccaccgggtacggaagtacctccac
agccggctttgccagctcgctgatcgccggttatggcagtacgcagacagccggctataaaagtaccctcacggccggttacggcagtactcag
accgcagagtatggaagctcactcactgcgggctacggcagcactgcaacggccgggcaggacagttcattgatagccggctatggcagctccc
tgaccagcggaatcagaagttttctgacgcaggctatggcagtacgctgatcgccggacttcgcagcgttttgatcgccggttatggcagtagtc
ttacatcgggcgttcgcagcacgttgactgcgggttatggcagtaaccagattgcaagttacggcagctcgttgattgcaggccatgaaagcatt
caggtcgccggaaataaaagcatgctgatcgccggcaagggcagctcgcagacagcaggttttcgcagcacgctgattgccggtgcgggcagt
gtacaactggcgggtgatcgcagccggttgattgccggtgcagacagtaatcagaccgcgggtgaccgcagcaaactgctggccggtaataac
agttatctgactgccggcgatagaagcaaactgaccggcgggcatgactgcaccctgatggcgggagaccaaagcagattgaccgctggtaag
aacagtgtcttgacggcaggcgctcgtagcaaactgattggcagtgaaggctcgacgctctcggctggagaagactccatacttattttcaggct
ctgggacgggaagaggtacaggcaactggtcgccagaacgggtgagaacggtgttgaggccgacataccgtattacgtgaacgaagatgacg
atattgtcgataaacccgacgaggacgatgactggatagaggtaaagtag

Figure 9

*Sequence of -NINP of inaZ gene (Including stop codon) (SEQ ID NO:2)*

agtaaacaccctgccggtgacgacgccgcagaatgtgcagaccgccacttacggcagcacgttgagtggcgacaatcacagtcgtctgattgcc

ggttatggcagtaacgagaccgctggcaaccacagtgatctaattgccggttatggaagtacaggcaccgccggctccgacagctggctggtcg

ctggctatggaagcacccagaccgccggtggggacagcgcgctgacagcgggttacggcagcacccagaccgcccgcgaaggcagcaacctg

acggcagggtacggcagcaccggcacggcaggctcggacagttcgctgatcgccggttacggcagtactcagacttcgggcgggggacagctca

ctcacagcgggttacggcagcacgcaaacggctcaggaaggcagcaatctcaccgctgggtatggcagcaccggcacggcaggctcggacag

ctcgttgatcgccggttatggcagtacacaaacctcgggaggcgacagttcgctgaccgcgggctacggcagtacgcagacggcccaggaggg

cagcaatctgacggcggggtacggcagcacgggtacagcaggtgtcgacagctctctgatcgcgggatacggcagcacgcagacctcgggaa

gtgacagcgccctgaccgcaggctatggcagcacgcaaacggcccaggaaggcagcaatctcactgctgggtatggcagcaccggcacggca

ggttccgacagctcgctgatcgccggttacggcagcacgcaaacctcgggcagtgacagctcgctcacggcggggtacggcagtacgcagacg

gctcaggaaggcagcaatctgacggcggggtacggcagcacgggtacagcaggtgtcgacagttcgttgatcgccggatatggcagcacgcag

acctcgggaagtgacagtgcgctgacagcgggttacggcagcacgcaaacggcccaggaaggcagcaacctgacggcgggctacggcagca

ctggcacggcaggtgccgacagttcgttgatcgccggatatggcagcacgcagacgtcaggcagcgaaagttcgcttaccgcaggctatggcag

tacccagactgcccgtgagggcagcaccctgacggccggatatggcagtaccggaacagctggcgctgacagctcgctgatcgccggttacggc

agcacgcaaacctcgggcagtgaaagctcgctcacggcaggttatggcagtacccagaccgcacagcagggcagcgtactcacatcaggctat

ggcagtacgcaaacggccggggctgccagtaacctcaccaccggttacggaagtacaggtaccgcaggtcacgagagtttcatcattgcgggtt

atggaagtacacagacagcgggccacaaaagtatcctgaccgctggttatggcagtactcagacggccagggacggtagcgacctgattgcgg

gctatggcagtacgggaaccgcaggctcgggcagttcgctgatcgcaggttatggcagcacccagaccgcgagttacagaagcatgctgaccg

ccggttatggcagtacccagaccgccagagaacacagcgaccttgtcacaggctatggcagcacttcaacggcagggtcaaacagttcgctgat

cgccggctatggaagcactcagacggcgggcttcaaaagcatactgaccgccggttacggcagtacccagacggcacaggagcgcagcgacc

tggtcgcaggctacggaagcacgtcgactgcgggctattccagttccttgatcgccggctatggcagcacgcagacggcaggctacgaaagcac

gttgaccgccggttacggcagtacgcaaaccgctcaggaaaacagctcgctcaccacaggttacggaagtacttctactgcgggctattccagct

cgctcatcgcgggttacggcagtacgcaaacggcaggctacgagagcacgttgaccgccggttacggtagtacgcaaaccgcgaaggagcgc

agtgatctggtgacaggttatggaagtacctccaccgccggctatgcgagctcgctgattgcgggttatggcagcacgcagactgcgggttatga

gagcacgttgaccgccggttacggcagcacgcaaaccgcacaggaaaacagctcgctcaccaccgggtacggaagtacctccacagccggctt

tgccagctcgctgatcgccggttatggcagtacgcagacagccggctataaaagtaccctcacggccggttacggcagtactcagaccgcagag

tatggaagctcactcactgcgggctacggcagcactgcaacggccgggcaggacagttcattgatagccggctatggcagctccctgaccagcg

gaatcagaagttttctgacggcaggctatggcagtacgctgatcgccggacttcgcagcgttttgatcgccggttatggcagtagtcttacatcgg

gcgttcgcagcacgttgactgcgggttatggcagtaaccagattgcaagttacggcagctcgttgattgcaggccatgaaagcattcaggtcgcc

ggaaataaaagcatgctgatcgccggcaagggcagctcgcagacagcaggttttcgcagcacgctgattgccggtgcgggcagtgtacaactg

gcgggtgatcgcagccggttgattgccggtgcagacagtaatcagaccgcgggtgaccgcagcaaactgctggccggtaataacagttatctga

ctgccggcgatagaagcaaactgaccggcgggcatgactgcaccctgatggcgggagaccaaagcagattgaccgctggtaagaacagtgtct

tgacggcaggcgctcgtagcaaactgattggcagtgaaggctcgacgctctcggctggagaagactccatacttattttcaggctctgggacggg

aagaggtacaggcaactggtcgccagaacgggtgagaacggtgttgaggccgacataccgtattacgtgaacgaagatgacgatattgtcgat

aaacccgacgaggacgatgactggatagaggtaaagtag

Figure 10

**A :** *InaZ Protein sequence – Original (SEQ ID NO:14)*

```
MNLDKALVLRTCANNMADHCGLIWPASGTVESRYWQSTRRHENGLVGLLWGAGTSAFLSV
HADARWIVCEVAVADIISLEEPGMVKFPRAEVVHVGDRISASHFISARQADPASTSTSTL
TPMPTAIPTPMPAVASVTLPVAEQARHEVFDVASVSAAAAPVNTLPVTTPQNVQTATYGS
TLSGDNHSRLIAGYGSNETAGNHSDLIAGYGSTGTAGSDSWLVAGYGSTQTAGGDSALTA
GYGSTQTAREGSNLTAGYGSTGTAGSDSSLIAGYGSTQTSGGDSSLTAGYGSTQTAQEGS
NLTAGYGSTGTAGSDSSLIAGYGSTQTSGGDSSLTAGYGSTQTAQEGSNLTAGYGSTGTA
GVDSSLIAGYGSTQTSGSDSALTAGYGSTQTAQEGSNLTAGYGSTGTAGSDSSLIAGYGS
TQTSGSDSSLTAGYGSTQTAQEGSILTAGYGSTGTAGVDSSLIAGYGSTQTSGSDSALTA
GYGSTQTAQEGSNLTAGYGSTGTAGADSSLIAGYGSTQTSGSESSLTAGYGSTQTAREGS
TLTAGYGSTGTAGADSSLIAGYGSTQTSGSESSLTAGYGSTQTAQQGSVLTSGYGSTQTA
GAASNLTTGYGSTGTAGHESFIIAGYGSTQTAGHKSILTAGYGSTQTARDGSDLIAGYGS
TGTAGSGSSLIAGYGSTQTASYRSMLTAGYGSTQTAREHSDLVTGYGSTSTAGSNSSLIA
GYGSTQTAGFKSILTAGYGSTQTAQERTSLVAGYGSTSTAGYSSSLIAGYGSTQTAGYES
TLTAGYGSTQTAQENSSLTTGYGSTSTAGYSSSLIAGYGSTQTAGYESTLTAGYGSTQTA
QERSDLVTGYGSTSTAGYASSLIAGYGSTQTAGYESTLTAGYGSTQTAQENSSLTTGYGS
TSTAGFASSLISGYGSTQTAGYKSTLTAGYGSTQTAEYGSSLTAGYGSTATAGQDSSLIA
GYGSSLTSGIRSFLTAGYGSTLIAGLRSVLIAGYGSSLTSGVRSTLTAGYGSNQIASYGS
SLIAGHESIQVAGNKSMLIAGKGSSQTAGFRSTLIAGAGSVQLAGDRSRLIAGADSNQTA
GDRSKLLAGNNSYLTAGDRSKLTGGHDCTLMAGDQSRLTAGKNSVLTAGARSKLIGSEGS
TLSAGEDSILIFRLWDGKRYRQLVARTGENGVEADIPYYVNEDDDIVDKPDEDDDWIEVK
```

**B:** *Sequenced InaZ protein used– Clone C4 (SEQ ID NO: 15)*
```
MNLDKALVLRTCANNMADHCGLIWPASGTVESRYWQSTRRHENGLVGLLWGAGTSAFLSV
HADARWIVCEVAVADIISLEEPGMVKFPRAEVVHVGDRISASHFISARQADPASTSTSTL
TPMPTAIPTPMPAVASVTLPVAEQARHEVFDVASVSAAAAPVNTLPVTTPQNVQTATYGS
TLSGDNHSRLIAGYGSNETAGNHSDLIAGYGSTGTAGSDSWLVAGYGSTQTAGGDSALTA
GYGSTQTAREGSNLTAGYGSTGTAGSDSSLIAGYGSTQTSGGDSSLTAGYGSTQTAQEGS
NLTAGYGSTGTAGSDSSLIAGYGSTQTSGGDSSLTAGYGSTQTAQEGSNLTAGYGSTGTA
GVDSSLIAGYGSTQTSGSDSALTAGYGSTQTAQEGSNLTAGYGSTGTAGSDSSLIAGYGS
TQTSGSDSSLTAGYGSTQTAQEGSNLTAGYGSTGTAGVDSSLIAGYGSTQTSGSDSALTA
GYGSTQTAQEGSNLTAGYGSTGTAGADSSLIAGYGSTQTSGSESSLTAGYGSTQTAREGS
TLTAGYGSTGTAGADSSLIAGYGSTQTSGSESSLTAGYGSTQTAQQGSVLTSGYGSTQTA
GAASNLTTGYGSTGTAGHESFIIAGYGSTQTAGHKSILTAGYGSTQTARDGSDLIAGYGS
TGTAGSGSSLIAGYGSTQTASYRSMLTAGYGSTQTAREHSDLVTGYGSTSTAGSNSSLIA
GYGSTQTAGFKSILTAGYGSTQTAQERSDLVAGYGSTSTAGYSSSLIAGYGSTQTAGYES
TLTAGYGSTQTAQENSSLTTGYGSTSTAGYSSSLIAGYGSTQTAGYESTLTAGYGSTQTA
KERSDLVTGYGSTSTAGYASSLIAGYGSTQTAGYESTLTAGYGSTQTAQENSSLTTGYGS
TSTAGFASSLIAGYGSTQTAGYKSTLTAGYGSTQTAEYGSSLTAGYGSTATAGQDSSLIA
GYGSSLTSGIRSFLTAGYGSTLIAGLRSVLIAGYGSSLTSGVRSTLTAGYGSNQIASYGS
SLIAGHESIQVAGNKSMLIAGKGSSQTAGFRSTLIAGAGSVQLAGDRSRLIAGADSNQTA
GDRSKLLAGNNSYLTAGDRSKLTGGHDCTLMAGDQSRLTAGKNSVLTAGARSKLIGSEGS
TLSAGEDSILIFRLWDGKRYRQLVARTGENGVEADIPYYVNEDDDIVDKPDEDDDWIEVK
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **J. KASSMANNHUBER et al.** *Atmos. Chem. Phys. Discuss*
- **J. KASSMANNHUBER et al.** *Bioengineered*, 2017, vol. 8 (5), 488-500
- **J. S. H. LORV et al.** *Scientifica*, vol. 2014, 1-20
- **HENRICH et al.** *Mol. Gen. Genet.*, 1982, vol. 185 (3), 493-497
- **SZOSTAK et al.** *Research in Microbiology*, 1990, vol. 141 (7-8), 1005-1007
- **J. KASSMANNHUBER et al.** Functional Display of Ice Nucleation Protein InaZ on the surface of Bacterial Ghosts. *Bioengineering*, 2017